# NEUE EUROPÄISCHE PATENTSCHRIFT

(11) **EP 0 798 039 B2**
(45) Veröffentlichungstag und Bekanntmachung des Hinweises auf die Entscheidung über den Einspruch: **09.01.2008**
(45) Hinweis auf die Patenterteilung: 18.06.2003
(21) Anmeldenummer: 97104966.3
(22) Anmeldetag: 24.03.1997
(51) Int. Cl.: B01J 19/24, B01J 8/22, B01J 8/20

(54) **Verfahren zur Durchführung von Stoffumwandlungen mit in Flüssigkeiten suspendierten Katalysatoren**
Method for carrying out the conversion of substances using catalysts suspended in liquids
Procédé pour la mise en oeuvre de la transformation de substances au moyen de catalyseurs en suspension dans des liquides

(30) Priorität: 26.03.1996 DE 19611976
(43) Veröffentlichungstag der Anmeldung: 01.10.1997
(73) Patentinhaber: BASF Aktiengesellschaft, 67056 Ludwigshafen (DE)
(72) Erfinder: Kaibel, Gerd, Dr., 68623 Lampertheim (DE); Bröcker, Franz-Josef, Dr., 67061 Ludwigshafen (DE); Stroezel, Manfred, 68549 Ilvesheim (DE)
(74) Vertreter: Isenbruck, Günter

(56) Entgegenhaltungen:
- EP-A- 0 070 330
- EP-A- 0 230 971
- EP-A- 0 606 964
- DE-B- 1 272 292
- DE-C- 938 252
- FI-A- 864 971
- NL-A- 8 702 882
- US-A- 4 847 016
- A.Ermakova et al., International Chemical Engineering, 1979, Vol. 19, 174-177
- L. Rusnac et al., Revista de Chimie (Bukarest) 41 (1990), 597-601
- Abstract zu D2 in Verfahrenstechnische Berichte 1991 (40):32
- Ullmann's Encyclopedia of Industrial Chemistry, 5th ed.1992,Vol.B4,568-571
- Abstract von D2 in Verfahrenstechnische Berichte 1991(40):32
- Auszug aus Prospekt d/23.27.06 - VI.86-60 der Firma Sulzer AG

## Beschreibung

Die vorliegende Erfindung bezieht sich auf Verfahren zur Durchführung von katalytischen Reaktionen, bei denen ein Katalysator in einer flüssigen Phase suspendiert ist. Diese Phase kann bereits ein flüssiges oder suspendiertes festes Reaktionsedukt enthalten. Häufig ist auch eine Gasphase vorhanden, aus der Reaktionsedukte in die flüssige Phase eingelöst werden. Typische Reaktionen dieser Art sind Oxidationen und Hydrierungen.

Zur Durchführung derartiger Reaktionen eignen sich unter anderem Blasensäulen und Rührbehälter. Die Technologie für Suspensionsreaktionen ist in Ullmanns Encyclopädie der technischen Chemie, 4. Auflage, 1973, Band 3, Seiten 494 bis 518, ausführlich beschrieben. Grundproblem derartiger Reaktionen ist die ausreichende Kontaktierung der Reaktionsedukte durch die Katalysatorpartikel, die in der Flüssigphase suspendiert sind.

Suspensionsreaktoren benötigen die Zufuhr mechanischer Energie, die beispielsweise durch Rührwerke, Düsen oder aufsteigende Gasblasen eingebracht wird, um die Feststoffteilchen zu suspendieren. Eine Erhöhung dieser mechanischen Energiezuführ über den zur Suspendierung erforderlichen Betrag hinaus führt aber zu keiner nennenswerten Verbesserung des Stoffübergangs zwischen der Flüssigkeit und den suspendierten Feststoffteilchen, da die erzielbare Relativgeschwindigkeit die Sedimentationsgeschwindigkeit nur unwesentlich übertrifft. Um diese Relativgeschwindigkeit zu erhöhen, wurde die Verwendung von Katalysatorpartikeln mit größeren Korngrößen (1 bis 10 mm), insbesondere in Fließbett- und Wirbelschichtverfahren, vorgeschlagen. Diese größeren Teilchen besitzen gegenüber der umgebenden Flüssigkeit zwar die gewünschte Relativgeschwindigkeit, ihre geringere volumenbezogene Oberfläche begrenzt aber andererseits wieder den Stoffumsatz. Beide Effekte kompensieren sich häufig, so daß das Problem der Erhöhung des Stoffumsatzes nicht gelöst wird.

Höhere Relativgeschwindigkeiten können lediglich bei Aufgabe der Suspensionsfahrweise in Festbettreaktoren erzielt werden. Bei dem Stand der Technik sind Reaktoren, beschrieben wobei statt Fastbetten mit suspendierten katalysatorpartikeln Bleche oder Gewebe mit Katalysatormaterial beschichtet werden, die von der flüssigen bzw. von der gasförmigen Phase angeströmt werden. Derartige Reaktoren werden in EP 068 862, EP 201 614 und EP 448 884 beschrieben. Ein Nachteil dieser Reaktoren besteht allerdings darin, daß das Katalysatormaterial nur durch Ersatz der beschichteten Bauteile erneuert werden kann. Das ist aufwendig und führt, wenn die Kontaminierung des Katalysatormaterials durch unreine Reaktionsedukte droht, zu aufwendigen Vorsichtsmaßnahmen, wie etwa der Hochreinigung der Ausgangsstoffe.

Aufgabe der vorliegenden Erfindung ist somit die Bereitstellung eines Verfahrens zur Durchführung von katalytischen Suspensionsreaktionen der beschriebenen Art mit einer erhöhten Raum-Zeit-Ausbeute.

Diese Aufgabe wird durch das in den Ansprüchen beschriebene Verfahren gelöst. Bei diesem Verfahren zur Durchführung von katalytischen Reaktionen in einem Reaktor, der eine Flüssigphase enthält, in der mindestens ein Katalysator suspendiert ist, wobei suspendierte Katalysatorpartikel mit einer mittleren korngrösse von 0.0001 bis 2mm, vorzugsweise von 0.001 bis 0.1mm, besonders bevorzugt von 0.005 bis 0.05 mm verwendet werden, und der zusätzlich eine Gasphase wird die Flüssigphase und enthält die Gasphase, zumindest teilweise, d.h. also zumindest mit einem Teil ihres Volumens oder auf einem Teil ihres Weges durch eine Vorrichtung mit Öffnungen oder Kanälen in dem Reaktor geführt, deren hydraulischer Durchmesser 0,5 bis 3 mm, vorzugsweise 1 bis 3 mm, beträgt. Reaktionsedukte können dabei sowohl als Flüssigkeit, als Feststoff oder als Gas eingebracht und in der Flüssigkeit gelöst werden. Im Rahmen der vorliegenden Erfindung spielt es grundsätzlich keine Rolle, in welchem Aggregatzustand die Reaktionsedukte zugeführt werden. Der hydraulische Durchmesser ist als Quotient des 4-fachen Querschnitts der Öffnung und deren Umfang definiert. Die Wahl der im Einzelfall idealen Kanalbreite hängt vor allem von der Viskosität der hindurchgeleiteten Flüssigkeit, der Größe der suspendierten Teilchen und der Art der Gasphase ab. Die Kanalbreiten müssen umso größer sein, je viskoser die Flüssigkeit ist. Bei Flüssigkeiten mit dynamischen Viskositäten zwischen 10x10⁻⁵ und 200x10⁻⁵ Ns/m² sind hydraulische Durchmesser im Bereich von 1 bis 3 mm optimal.

Bei dem erfindungsgemäßen Verfahren führen die Katalysatorteilchen gegenüber der Flüssigphase eine verstärkte Relativbewegung aus, weil sie in den engen Öffnungen und Kanälen eine Abbremsung gegenüber der umgebenden Flüssigkeit erfahren. Diese Abbremsung kann durch Kollisionen mit den Kanalwandungen ebenso verursacht werden wie durch kurzes Festhalten der Teilchen an rauhen Wandflächen. Bei dem erfindungsgemäßen Verfahren werden suspendierte Katalysatorpartikel mit einer mittleren Korngröße von 0,0001 bis 2 mm, besonders bevorzugt von 0,001 bis 0,1 mm, weiter bevorzugt von 0,005 bis 0,05 mm, verwendet . Diese Partikel führen mit ihrer hohen volumenbezogenen Oberfläche zu guten Resultaten, denn sie können infolge der Durchleitung durch die engen Einbauten Relativbewegungen gegenüber der Flüssigkeit ausführen. Im Ergebnis können deutlich höhere Raum-Zeit-Ausbeuten erzielt werden. Dabei zeigte sich im Versuch, daß bereits geringe Relativbewegungen der Katalysatorteilchen bzw. eine Abbremsung eines nur kleinen Teils der Katalysatorteilchen zu einer Beschleunigung der Reaktion führen.

Die Vorrichtung mit Öffnungen oder Kanälen zur Durchführung der Eduktphase besteht in einem Packungselement, wie es grundsätzlich, d.h. seiner geometrischen Form nach, bereits aus der Destillations- oder Extraktionstechnik bekannt ist, bestehen. Derartige Packungselemente, die den Vorteil eines geringen Druckverlusts bieten, sind zum Beispiel Drahtgewebepackungen der Bauart Montz A3 und Sulzer BX, DX und EX. Für Zwecke der vorliegenden Erfindung haben die Packungen jedoch grundsätzlich einen wesentlich, regelmäßig um den Faktor 2 bis 10 kleineren hydraulischen Durchmesser als vergleichbare Einbauten im Bereich der Destillations- oder Extraktionstechnik. Drahtgewebepackungen sind besonders vorteilhaft. Das beruht vermutlich darauf, daß ein Teil der Suspension nicht den gebildeten Kanälen folgt, sondern durch das Gewebe hindurchtritt. Statt Gewebepackungen können aber im Rahmen der vorliegenden Erfindung auch Packungen aus anderen gewebten, gewirkten oder gefilzten flüssigkeitsdurchlässigen Materialien verwendet werden. Bei weiteren geeigneten Packungen werden ebene Bleche, bevorzugt ohne Perforationen oder andere größere Öffnungen, eingesetzt, beispielsweise entsprechend den Bauarten Montz B1 oder Sulzer Mellapak. Vorteilhaft sind auch Packungen aus Streckmetall, wie zum Beispiel Packungen des Typs Montz BSH. Auch dabei müssen Öffnungen, wie etwa Perforationen, entsprechend klein gehalten werden. Entscheidend für die Eignung einer Packung im Rahmen der vorliegenden Erfindung ist nicht deren Geometrie, sondern die für die Stromführung entstehenden Öffnungsgrößen bzw. Kanalbreiten in der Packung.

Bevorzugt ist ein Verfahren, bei dem die Flüssigphase und die Gasphase durch Öffnungen oder Kanäle geführt wird, deren Wandmaterialien Oberflächenrauhigkeiten im Bereich des 0,1- bis 10-fachen, vorzugsweise des 0,5- bis 5-fachen, der mittleren Korngröße der suspendierten Katalysatorteilchen aufweisen. Die Aufrauhung der Öffnungen bzw. Kanalwände bewirkt eine besonders gute Abbremsung und damit Relativbewegung der suspendierten Katalysatorteilchen. Diese werden an den Wandflächen vermutlich kurz festgehalten, so daß sie erst verzögert wieder in den Flüssigkeitsstrom eintreten. Dabei hängt die bevorzugte Rauhigkeit des verwendeten Materials im Einzelfall von der Größe der suspendierten Katalysatorpartikel ab.

In einem bevorzugten Verfahren wird die Flüssig/Gasphase durch Öffnungen oder Kanäle mit metallischen Wandmaterialien geführt, deren Oberflächen einen Mittenrauhwert Rₐ nach DIN 4768/1 von 0,001 bis 0,01 mm aufweisen. Solche metallischen Oberflächen können zum Beispiel durch thermische Behandlung von Stählen, zum Beispiel Kanthal, in Sauerstoffatmosphäre hergestellt werden. Wirksam im Sinne der vorliegenden Erfindung sind somit nicht nur makroskopische, sondern auch mikroskopische Rauhigkeiten.

Das Verfahren zeichnet sich dadurch aus, daß die Flüssigphase mit einer Leerrohrgeschwindigkeit von etwa 50 bis 300 m³/m²h, durch die Vorrichtung mit Öffnungen und Kanälen geführt wird. Vorzugsweise sind die Flüssigkeit mit einer Leerrohrgeschwindigkeit von 150 bis 200 m³/m²/h durch die Vorrichtung mit Öffnungen und Kanälen geführt. Die Leerrohrgeschwindigkeit der Gasphase beträgt vorzugsweise 5 bis 300 m³/m²h, besonders bevorzugt 100 bis 200 m³/m²h.

Für die Durchführung des beanspruchten Verfahrens wird ein Reaktor beschrieben.

Dieser Reaktor verfügt über Einrichtungen zur Zu- und Abführung einer Flüssigphase bzw. Suspension. Der Reaktor weist auch weitere Zu- und Abführeinrichtungen für eine Gasphase auf. Der Reaktor enthält mindestens eine Vorrichtung mit Öffnungen und Kanälen, die einen hydraulischen Durchmesser von 0,5 bis 3 mm, vorzugsweise von 1 bis 3 mm, aufweisen, wobei die Vorrichtung derart in dem Reaktor eingebaut und angeordnet ist, daß die Flüssigphase und die Gasphase während der Reaktion zumindest teilweise durch die Öffnungen und Kanäle geführt wird. Dadurch wird auch hier die oben beschriebene intensivere Relativbewegung der Flüssigphase zu den Katalysatorpartikeln erreicht.

Die vorliegende Erfindung kann in verschiedenen kontinuierlich oder diskontinuierlich betriebenen Reaktorbauformen wie Strahldüsenreaktoren, Blasensäulen, Rohrreaktoren, Rohrbündelreaktoren und Rührbehältern realisiert werden. Bei einem Rührbehälter können die erfindungsgemäßen Einbauten auch direkt auf der Rührerwelle befestigt sein und zumindest teilweise die Funktion eines Rührorgans übernehmen. Sie können auch zusätzlich als Strombrecher wirken. Die oben vorgestellten Einbauten füllen - abgesehen von der Anwendung in Rührbehältern - vorzugsweise, aber nicht notwendigerweise den gesamten Reaktor aus. Der Reaktor ist vorzugsweise eine vertikal stehend angeordnete Blasensäule, die bevorzugt im Gleichstrom von unten nach oben durchströmt wird. Ein anderer bevorzugter Reaktor ist ein heiz- oder kühlbarer Rohrbündelreaktor, bei dem die erfindungsgemäßen Einbauten in den einzelnen Rohren untergebracht sind. Der Reaktor wird vorzugsweise von unten nach oben durchströmt. Bevorzugt ist auch ein Spiral- oder Plattenwärmetauscher, der die entsprechenden Vorrichtungen enthält. Geeignet ist ebenso als Reaktor ein Rührbehälter, bei dem die Einbauten in die Strombrecher und/oder Rührorgane integriert sind.

Das suspendierte Katalysatormaterial kann mit Hilfe gebräuchlicher Techniken eingebracht und wieder abgetrennt werden (Sedimentation, Zentrifugation, Kuchenfiltration, Querstromfiltration).

Die Vorrichtung mit Öffnungen oder Kanälen besteht in einem Packungselement aus der Destillations- oder Extraktionstechnik. Dabei gilt das oben zum Verfahren Gesagte hier entsprechend.

Ein weiterer bevorzugter Reaktor zeichnet sich dadurch aus, daß die Öffnungen bzw. Kanäle der Vorrichtung Wandmaterialien mit Oberflächenrauhigkeiten aufweisen, die im Bereich des 0,1- bis 10-fachen, vorzugsweise des 0,5- bis 5-fachen der mittleren Korngröße der suspendierten Katalysatorteilchen liegen. Insbesondere können metallische Wandmaterialien verwendet werden, deren Oberflächen einen Mittenrauhwert Rₐ nach DIN 4768/1 von 0,001 bis 0,01 mm aufweisen.

Die Abtrennung der suspendierten Feststoffe erfolgt nach den üblichen Trennungsverfahren, wie zum Beispiel Kuchenfiltration oder Kerzenfiltration. Als besonders geeignet erwies sich bei kontinuierlicher Reaktionsführung die Querstromfiltration.

Die vorliegende Erfindung wird anhand der folgenden Figuren im Detail beschrieben. Dabei zeigen:
- Figur 1:: Versuchsaufbau für diskontinuierliche Reaktionsführung mit einem erfindungsgemäß modifizierten Blasensäulenreaktor
- Figur 2:: Versuchsaufbau für kontinuierliche Reaktionsführung mit einem erfindungsgemäß modifizierten Blasensäulenreaktor
- Figur 3:: Versuchsaufbau für kontinuierliche Reaktionsführung mit einem erfindungsgemäß modifizierten Rohrbündelreaktor

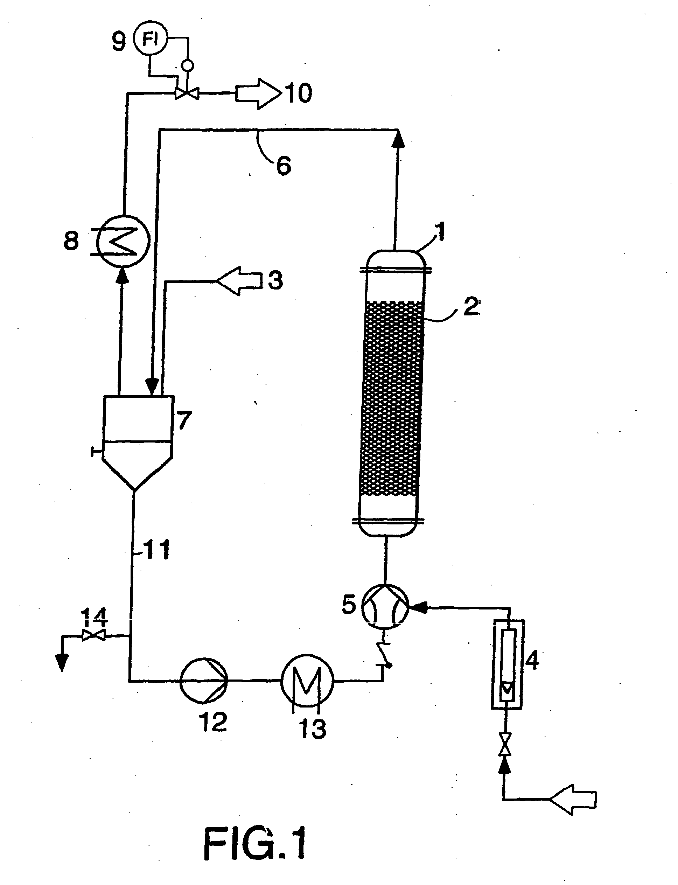

Figur 1 zeigt beispielhaft einen Versuchsaufbau mit einem diskontinuierlich betriebenen Blasensäulenreaktor 1, in dem entsprechend der vorliegenden Erfindung eine Gewebepackung 2 angeordnet ist, deren Geometrie mit der Destillationspackung Sulzer BX vergleichbar ist. Zur Durchführung der Reaktion wird zunächst ein flüssiger Reaktand mit suspendiertem Katalysator über die Befülleitung 3 eingebracht. Über die Anschlußleitung 4 wird ein gasförmiger Reaktand zugefahren und in einer Mischdüse 5 mit der umgepumpten Suspension vermischt und dieses Gemisch am unteren Ende des Reaktors 1 zugefahren. Die Suspension wird über die Leitung 6 zusammen mit Gas aus dem Reaktor ausgetragen und in das Abscheidegefäß 7 geleitet. Von dort wird das Gas über einen Abgaskühler 8 geleitet und über eine Druckhaltung 9 in die Abgasleitung 10 geführt. Die Suspension gelangt aus dem Abscheidegefäß 7 über die Leitung 11 in die Pumpe 12, den Wärmetauscher 13, die Mischdüse 5 und weiter in den Reaktor 1. Nach beendeter Reaktion wird die Suspension über die Entnahmeleitung 14 abgelassen.

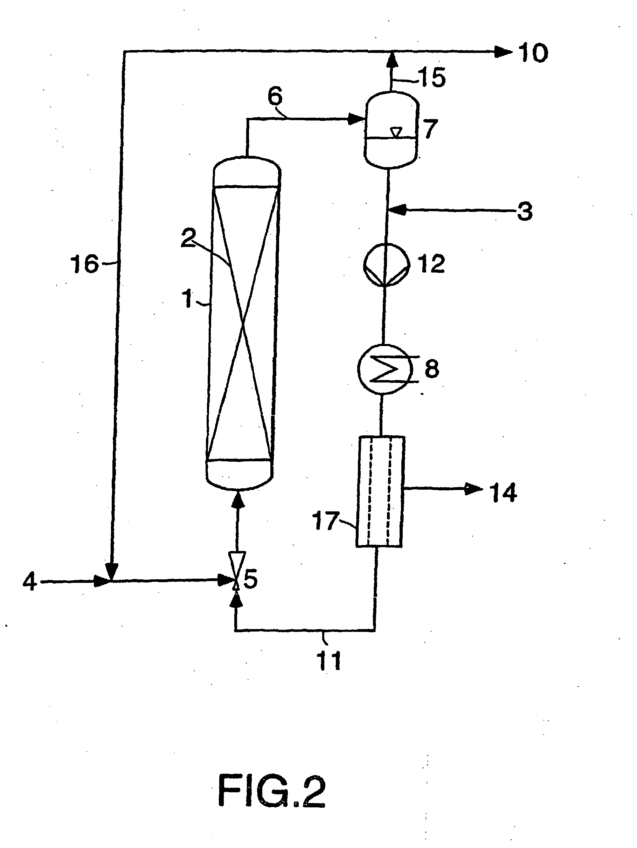

Figur 2 zeigt einen kontinuierlich betriebenen Reaktor 1 mit Packungen 2, der über die Leitungen 15 und 16 zusätzlich mit Kreisgas betrieben wird, das zusammen mit Frischgas 4 über die Mischdüse 5 in die im Kreis geführte Suspension 11 eingemischt wird. Der Reaktoraustrag wird über die Leitung 6 in das Abscheidegefäß 7 gefahren, in dem die Gasphase abgeschieden und über die Leitung 15 abgeführt wird. Von dieser Gasmenge wird zur Begrenzung der Aufpegelung von gasförmigen Verunreinigungen ein Teilstrom über die Leitung 10 entnommen und die verbleibende Restmenge über die Leitung 16 wieder in den Reaktor geführt. Über die Leitung 3 wird nur flüssiger Reaktand eingebracht. Der suspendierte Katalysator verbleibt in dem Reaktorsystem, indem er über ein Querstromfilter 17 zurückgehalten wird und nur katalysatorfreie Flüssigkeit 14 austritt und entnommen wird.

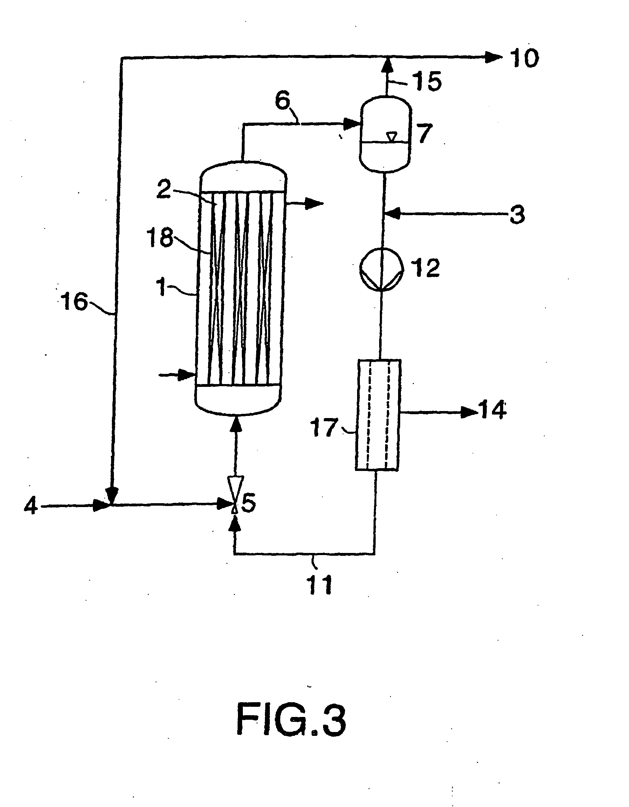

Figur 3 zeigt die bei schnell verlaufenden Reaktionen mit hoher Wärmetönung bevorzugt einzusetzende Ausführungsform, bei der der Reaktor 1 die Bauform eines Rohrbündelwärmetauschers aufweist und Rohrbündel 18 besitzt, in denen Drahtgewebefüllungen 2 angeordnet sind. Der in Figur 1 und 2 vorhandene Wärmetauscher 8 kann bei dieser Ausführungsform entfallen. Er kann beibehalten werden, um die Funktion eines Aufheizers zu Beginn der Reaktion zu übernehmen, wenn der Reaktor nur mit Kühlmittel beaufschlagt werden soll. Die Funktionen der Teile 4, 5, 6, 7, 10, 12, 14, 16 und 17 entsprechen denen in Figur 2.

### BEISPIELE

Die Vorteile der beschriebenen Erfindung werden im folgenden anhand von Beispielen zur Hydrierung von Hydrodehydrolinalool zu Hydrolinalool und weiter zu Tetrahydrolinalool beschrieben. Dabei wird die Dreifachbindung zunächst zur Doppelbindung und schließlich zur Einfachbindung hydriert.

Die Reaktion wurde einerseits in einem herkömmlichen Rührbehälter mit einem dreiflügeligen Begasungsrührer (1 l Volumen, 54 mm Rührerdurchmesser, 1000 U/min) und Stromstörern durchgeführt.

Andererseits wurde eine mit einer Gewebepackung bestückte Blasensäule (400 mm Höhe, 40 mm Durchmesser) entsprechend der vorliegenden Erfindung zur Hydrierung verwendet. Der Versuchsaufbau entsprach Figur 1. Die Geometrie der Packung entsprach einer handelsüblichen Gewebepackung vom Typ Sulzer BX, das Drahtgewebe war mit einer Drahtstärke von 0,112 mm jedoch feiner und die Spaltweiten waren enger. Die Knickbreite betrug 1 mm, der Neigungswinkel der Knicke gegen die Vertikale war 60°. Die volumenbezogene Oberfläche der Packung betrug 2200 m²/m³, wobei sich diese Angabe nur auf die geometrische Oberfläche der Gewebe analog zu ebenen Blechen bezieht. Die Flüssigkeit mit dem suspendierten Katalysator und das Gas wurde mit einer Leerrohrgeschwindigkeit von 175 m³/m²h von unten in den Reaktor mit der Packung eingebracht.

Die Reaktion erfolgte in beiden Fällen diskontinuierlich unter einem Druck von 1,1 bar. Als Katalysator wurde ein mit ZnO dotierter Palladium-Katalysator auf einem CaCO₃-Trägermaterial verwendet, der eine mittlere Korngröße um 0,01 mm aufwies. Bei den Vergleichsversuchen im Rührbehälter betrug die Menge an eingesetztem Hydrodehydrolinalool 650 g, bei den Versuchen mit dem erfindungsgemäßen Reaktor betrug sie 840 g. Die Hydrierung erfolgte bei 80 °C.

### Beispiel 1

Gepackte Blasensäule mit 0,15 Gew-% Katalysator. Die Hydrierung der Dreifachbindung war nach 1,75 Stunden, die der Doppelbindung nach 4 Stunden abgeschlossen.

### Beispiel 2

Gepackte Blasensäule mit 0,31 Gew-% Katalysator. Die Hydrierzeiten stimmten ungefähr mit denen aus Beispiel 1 überein.

### Vergleichsbeispiel 1

Rührbehälter mit 0,15 Gew-% Katalysator. Die Hydrierung der Dreifachbindung war nach 5 Stunden, die der Doppelbindung nach 11 Stunden abgeschlossen.

### Vergleichsbeispiel 2

Rührbehälter mit 0,31 Gew-% Katalysator. Die entsprechenden Hydrierzeiten betrugen 3,5 und 7 Stunden.

### Vergleichsbeispiel 3

Rührbehälter mit 0,77 Gew-% Katalysator. Die Hydrierzeiten verkürzten sich auf 3 und 5,5 Stunden.

Aus den Vergleichsversuchen ergibt sich, daß bei einem konventionellen Reaktor die Raum-Zeit-Ausbeute mit der eingebrachten Katalysatormenge steigt. Daraus folgt, daß die Reaktionsgeschwindigkeit stoffübergangskontrolliert ist. Bei Anwendung des erfindungsgemäßen Verfahrens bleiben die Reaktionszeiten dagegen unverändert, wenn die zugesetzte Katalysatonnenge erhöht wird. Das zeigt, daß die Reaktionsgeschwindigkeit nicht mehr stoffübergangskontrolliert ist. Die maximal mögliche Reaktionsgeschwindigkeit wird in den erfindungsgemäßen Versuchen durch den Stoffübergang von der Gasphase in die Flüssigkeit bestimmt. Sie wird bereits bei relativ kleinen Katalysatorkonzentrationen erreicht.

## Patentansprüche

1. Verfahren zur Durchführung von katalytischen Reaktionen in einem Reaktor, der eine Flüssigphase enthält, in der mindestens ein Katalysator suspendiert ist, und der zusätzlich eine Gasphase enthält, **dadurch gekennzeichnet, dass** die Flüssigphase und die Gasphase zumindest teilweise durch eine Vorrichtung mit Öffnungen oder Kanälen in dem Reaktor geführt wird, deren hydraulischer Durchmesser 0,5 bis 3 mm, vorzugsweise 1 bis 3 mm beträgt, dass suspendierte Katalysatorpartikel mit einer mittleren Korngröße von 0,0001 bis 2 mm, vorzugsweise von 0,001 bis 0,1 mm, besonders bevorzugt von 0,005 bis 0,05 mm, verwendet werden und dass die Flüssigphase mit einer Leerrohrgeschwindigkeit von 50 bis 300 m³/m²/h durch die Vorrichtung mit Öffnungen und Kanälen geführt wird,
dass als Vorrichtung mit Öffnungen oder Kanälen ein Packungselement, wie es grundsätzlich aus der Destillations- oder Extraktionstechnik bekannt ist, verwendet wird, und eine gleichzeitig anwesende Gasphase mit einer Leerrohrgeschwindigkeit von vorzugsweise 5 bis 300 m³/m²h, besonders bevorzugt von 100 bis 200 m³/m²h, durch die Vorrichtung mit Öffnungen oder Kanälen geführt wird.

2. Verfahren nach Anspruch 1, **dadurch gekennzeichnet, dass** die Flüssigphase und die Gasphase zumindest teilweise durch Öffnungen oder Kanäle geführt wird, deren Wandmaterialien Oberflächenrauhigkeiten im Bereich des 0,1- bis 10-fachen, vorzugsweise des 0,5- bis 5-fachen der mittleren Korngröße der suspendierten Katalysatorteilchen aufweisen.

3. Verfahren nach einem der vorgehenden Ansprüche, **dadurch gekennzeichnet, dass** die Flüssigphase und die Gasphase durch Öffnungen oder Kanäle mit metallischen Wandmaterialien geführt wird, deren Oberflächen einen Mittenrauhwert Rₐ nach DIN 4768/1 von 0,001 bis 0,01 mm aufweisen.

## Claims

1. A process for carrying out catalytic reactions in a reactor which contains a liquid phase in which at least one catalyst is suspended, and which additionally contains a gas phase, wherein the liquid phase and the gas phase are fed, at least partly, through an apparatus having orifices or channels in the reactor, the hydraulic diameter of which orifices or channels is from 0.5 to 3 mm, preferably from 1 to 3 mm,
suspended catalyst particles having a mean particle size of from 0.0001 to 2 mm, preferably from 0.001 to 0.1 mm, particularly preferably from 0.005 to 0.05 mm, are used, and wherein the liquid phase is fed through the apparatus having orifices and channels at en empty tube velocity of from 50 to 300 m³/m²h, wherein a packing element as known in principle from distillation or extraction technology is used as the apparatus having orifices or channels, and a gas phase simultaneously present is fed through the apparatus with orifices or channels at an empty tube velocity of, preferably, from 5 to 300, particularly preferably from 100 to 200, m³/m²h.

2. A process as claimed in claim 1, wherein the liquid phase and the gas phase are fed, at least partly, through orifices or channels whose wall materials have surface roughnesses of from 0.1 to 10, preferably from 0.5 to 5, times the mean particle size of the suspended catalyst particles.

3. A process as claimed in any of the preceding claims, wherein the liquid phase and the gas phase are fed through orifices or channels having metallic wall materials whose surfaces have a center line average value Rₐ according to DIN4768/1 or from 0.0001 to 0.01 mm.

## Revendications

1. Procédé pour la mise en oeuvre de réactions catalytiques dans un réacteur, qui contient une phase liquide, dans laquelle au moins un catalyseur est mis en suspension, et qui contient en supplément une phase gazeuse, **caractérisé en ce que** la phase liquide et éventuellement la phase gazeuse sont guidées dans le réacteur au moins partiellement au travers d'un dispositif présentant des ouvertures ou canaux dont le diamètre hydraulique est de 0,5 a 3 mm, de préférence de 1 à 3 mm, **en ce que** des particules de catalyseur en suspension ayant une taille granulaire moyenne de 0,0001 à 2 mm, de préférence de 0,001 à 0,1 mm, particulièrement avantageusement de 0,005 à 0,05 mm, sont utilisées, et **en ce que** la phase liquide est guidée au travers du dispositif à ouvertures ou canaux à une vitesse en tube vide de 50 à 300 m³/m²h, **en ce que**, comme dispositif présentant des ouvertures ou canaux, on utilise un élément de garnissage, tel qu'il est connu en principe dans la technique de distillation ou d'extraction, et une phase gazeuse simultanément présente ayant une vitesse en tube vide de préférence de 5 à 300 m³/m²h, particulièrement avantageusement de 100 à 200 m³/m²h, est guidée au travers du dispositif à ouvertures ou canaux.

2. Procédé suivant le revendication 1, **caractérise en ce que** la phase liquide et la phase gazeuse sont guidées au moins partiellement au travers d'ouvertures ou de canaux dont les matiéres de paroi présentent des rugosités de surface de l'ordre de 0,1 à 10 fois, de préférence de 0,5 à 5 fois, la taille granulaire moyenne des particules de catalyseur en suspension.

3. Procédé suivant l'une des revendications précédentes, **caractérisé en ce que** la phase liquide et la phase gazeuse sont guidées au travers d'ouvertures ou de canaux présentant des matières de paroi métalliques dont les surfaces présentent une valeur de rugosité moyenne Rₐ selon DIN 4768/1 de 0,001 à 0,01 mm.
